# EUROPEAN PATENT APPLICATION

(11) **EP 4 151 243 A1**
(43) Date of publication of application: **22.03.2023**
(21) Application number: 21382835.3
(22) Date of filing: 15.09.2021
(51) Int. Cl.: A61L 11/00, A61B 50/39, A61M 5/32, B09B 3/00

(54) **CONTAINER FOR COLLECTING AND STERILIZING MEDICAL WASTE**

(71) Applicant: Trilderson, S.L., 31003 Santa Pola (Alicante) (ES)
(72) Inventor: LÓPEZ LÓPEZ, Manuel Jorge, 31003 Santa Pola (Alicante) (ES); NÚÑEZ GAGO, José Luis, 31003 Santa Pola (Alicante) (ES)
(74) Representative: Hoffmann Eitle

(57) **Abstract**

The present invention refers to a medical waste container (1), comprising: an upper base (2), a bottom base (3a) and a side wall (3b) joined to the bottom base (3a) to define the container interior (4) for containing waste, and at least one opening (5) for introducing waste into the container (1). The container (1) further comprises closing means for closing the at least one opening (5), and a pipe (8) placed inside the container (1) and arranged to fluidly communicate the interior of the container (1) with the exterior of the container (1), such that a disinfecting gas or liquid can circulate along the pipe (8) into the container (1). The at least one opening (5) is arranged relative to the pipe (8) such that waste introduced through the opening (5) would be received at the container interior. The container (1) of the invention features a simple design such that it can be manufactured at a low cost.

## Description

### Field and object of the invention

The present invention refers in general to containers for collecting infectious material like medical waste, especially needles, syringes, cannulas, scalpels or other utensils that are contaminated by viruses or bacteria.

An object of the invention is to provide a reusable container for collecting and sterilizing medical waste inside the container by means of an autoclave or similar process, wherein the container features a simple design such that it can be manufactured at a low cost.

### Background of the invention

Infectious medical waste like: needles, hypodermic syringes, cannulas, scalpels or other biofluid contaminated material, must be securely collected and handled for disposal to prevent infecting human operators and environment contamination.

In this technical field, there are different types of medical waste containers normally used in hospitals or other medical facilities to securely collect infectious waste, so that, once the containers are full of waste, they are transported to a disposal facility for the disinfection of the infected waste, in some cases by incineration of the container itself and its content.

Other type of containers are reusable, and are adapted to disinfect the waste material inside the container by means of pressurized steam, typically in an autoclave or similar equipment, such that container content is sterilized before the container is opened. In this way, when the waste is taken out of the container it is already sterilized, and infections in the personnel handling the waste are prevented. The desinfected waste is finally processed for its final disposal.

However, known containers of this type are based on pressure valves or other complex and expensive mechanisms.

One example of this type of containers is described in the Spanish Utility Model ES-1.256.284 U. The container described in this publication is suitable for autoclave sterilization for medical and hospital waste, such that the container has a lid and holes for the inlet of steam from an autoclave inside the container, and it is provided with a valve for sealing the holes, said valve having means of activation by temperature, which unblocks the holes when a certain temperature is reached. When the valve closes the holes, the container is completely closed.

The provision of this valve activated by temperature, renders the container expensive and unreliable because the valve deteriorates with time, causing malfunctions and an undesired repair costs.

### Summary of the invention

The present invention is defined in the attached independent claim, and satisfactorily solves the shortcomings of the prior art outlined above, by providing a medical waste container, that allows sterilization of its content inside the container by means of a disinfecting agent like steam or a liquid, in a passive manner, that is, without relying in valves of other mechanisms.

Therefore, an aspect of the invention refers to a medical waste container comprising at least one closable opening for introducing waste into the container, and a pipe placed inside the container and communicating, at atmospheric pressure, the exterior of the container with its interior, such that a disinfecting gas or liquid can circulate freely from the exterior of the container and along the pipe, into the container, that is, there is no valve or any other device place inside the pipe. The opening is arranged in the container relative to the pipe (namely, relative to an open free end of the pipe), such that infected objects inserted through the opening would fall by gravity and collected at the bottom of the container, that is, the objects cannot reach the exterior of the container through the pipe.

The above-described container, can be placed in an autoclave or similar equipment, such that pressurized steam can pass through the pipe and circulate into the container interior to sterilize inside the container the objects collected inside, without the participation of any pressure or temperature activated mechanism.

The container is formed by an upper base, a bottom base and a side wall joined to the bottom base to define a bucket for containing waste. The container comprises closing means for closing the at least one opening, such that when the opening is closed, waste cannot be extracted out of the container.

The pipe is a tubular body with any a cross-sectional shape, preferably cylindrical or squared, and it has two open ends, one open end is joined to the bottom base, to the upper base, or to the side wall, and the other end is a free end which is placed inside the container.

In a preferred example, the pipe is straight and has one end joined to the bottom base, and its free end is adjacent and placed below the upper base.

The at least one opening is arranged such that it does not overlap with the free end of the pipe so that waste would be collected by gravity at the bottom base of the container, in order to prevent that objects inserted through the opening could accidentally get into the pipe and from there get out of the container. Preferably, the at least one opening is provided in the upper base.

The upper base is movable with respect to the side wall, to allow extraction of the content of the container once it has been disinfected. The side wall has a free edge having a circumferential shape, and the upper base has a circular configuration that is couplable to the free edge of the side wall.

The container comprises locking means configured to be set in a locked position in which they attach the upper base to the side wall, and in a released position in which the upper base can be moved with respect to the side wall. Preferably, the locking means are accessible from outside the container through the pipe, to be set reversely either in the locked position or in the released position.

The closing means for closing the at least one opening, comprises a lid mounted on the upper base. Preferably, the lid has openings and it is rotatable on the upper base between a closed position in which the lid is overlapped with the opening closing it, and an open position in which the openings of the upper base and the openings of the lid are overlapped, such that the openings of the upper base are accessible for inserting waste into the container.

The locking means comprises first engaging means attached to the side wall, and second engaging means coupled or couplable with the upper base. The first and second engaging means, are configured to engage and disengage with each other to attach or release the upper base from the side wall.

In a preferred embodiment, the first engaging means are fixed to an internal surface of the side wall, and the second engaging means are coupled to the upper base and are rotatable with respect to the first engaging means. In addition, first and second engaging means are configured to engage and disengage with each other upon rotation of the second engaging means. The second engaging means are accessible from outside the container through the pipe to be rotated, when the pipe is straight and is connected to the bottom base.

Preferably, the upper base is disc-shaped and it has upper and lower surfaces. The lid is mounted on the upper surface of the upper base, and the second engaging means are mounted on the lower surface of the upper base.

The first engaging means includes hooks, and the second engaging means include an axis coupled to the upper base and two or more arms radially extending from the axis. The arms are provided with loops at their free ends, and are configured in a way that the hooks of the first engaging means can be inserted in the loops upon rotation of the second engaging means. Preferably, the pipe and the axis are co-axially arranged.

The container is complemented with a tool, configured to be inserted from outside the container through the pipe and reach the second engaging means. One end of the tool and the second engaging means, are configured to engage with each other such that the second engaging means can be rotated by rotating the tool.

The invention provides a reusable container for collecting and sterilizing medical waste inside the container by means of an autoclave or similar process. The container features a simple design, is reliable, and it can be manufactured at a low cost because it does not rely on active devices like valves or other expensive and unreliable mechanisms.

### Brief description of the drawings

Preferred embodiments of the invention are henceforth described with reference to the accompanying drawings, wherein:
Figure 1.- shows two exploded views in perspective of a preferred embodiment of the invention, from above (Figure 1A), and from below (Figure 1B).
Figure 2.- shows three views of the container of Figure 1 in an assembled arrangement, wherein Figure 2A is a top plan view, Figure 2B is an elevational cross-sectional view taken on plane D-D in Figure 2A, and Figure 2C is a perspective view of the representation of Figure 2B.

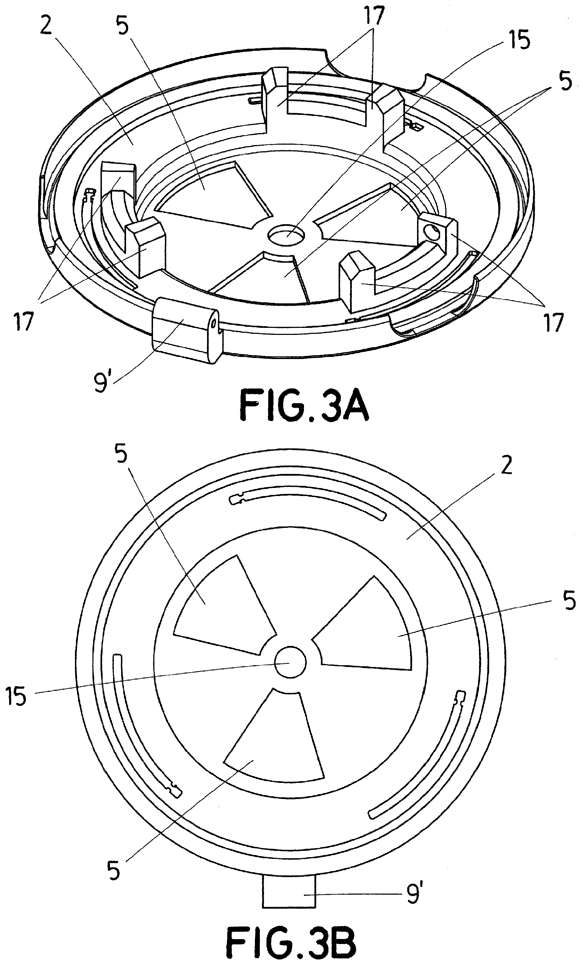

Figure 3.- shows the upper base, in a perspective view from below (Figure 3A), and in a top plan view (Figure 3B).
Figure 4.- shows a perspective view of the second engaging means in Figure 4A, and a bottom plan view in Figure 4B.

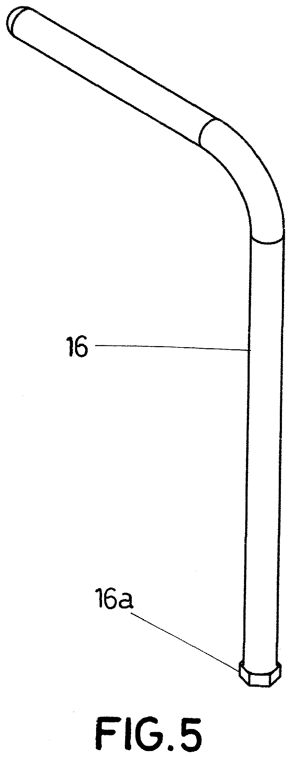

Figure 5.- is a perspective view of the tool for locking and unlocking the upper base of the container.

### Preferred embodiment of the invention

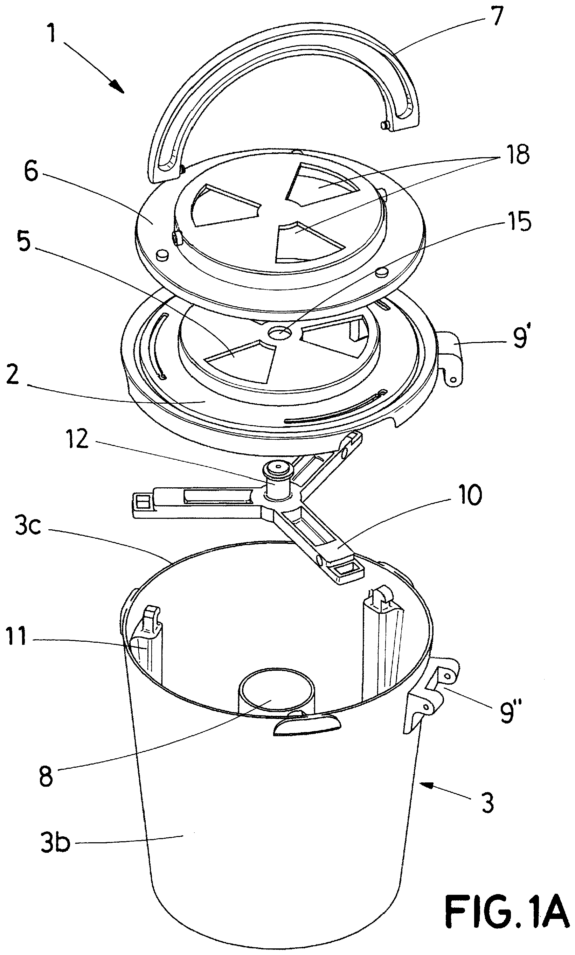

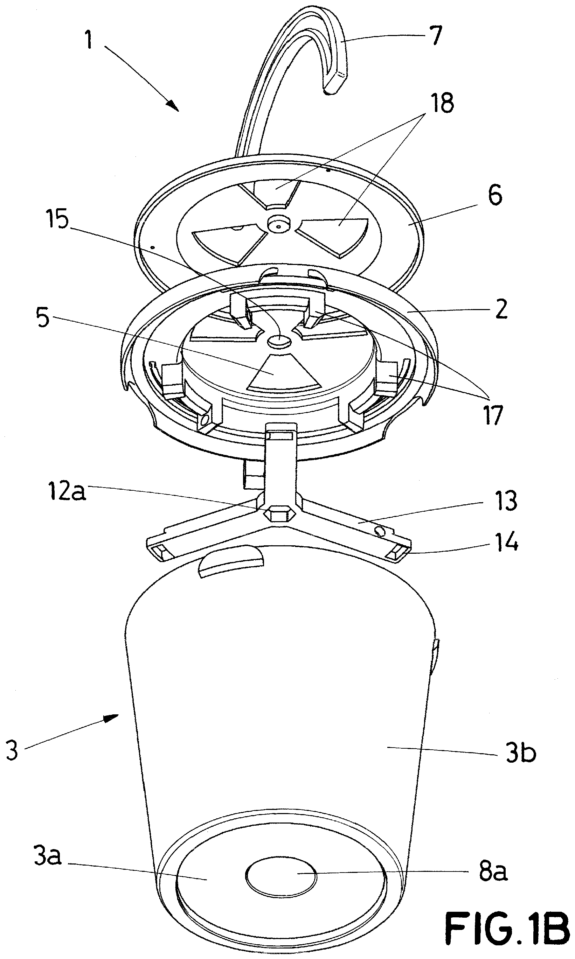

**Figures 1A****,** **1B** show a preferred embodiment of a medical waste container (1) according to the invention, that comprises an upper base (2), and a bucket (3) formed by a bottom base (3a) and a side wall (3b) joined to the bottom base (3a) to define the container interior (4) for containing waste.

The side wall (3b) has a free edge (3c) having a circumferential shape, such that the upper base (3c) has a circular configuration and it is couplable to the free edge (3c) of the side wall.

The container (1) comprises at least one opening (5) formed in the upper base (2), for introducing waste into the container. In the exemplary embodiment shown in the figures, there are three openings (5) equidistantly arranged.

In addition, the container (1) is provided with closing means for closing the at least one opening (5). Preferably, the closing means include a lid (6) which has openings (18) in correspondence with the openings (5) of the upper base (2). The lid (6) is mounted on the upper surface of the upper base (2), and rotatable with respect to the upper base (2) between a closed position in which the lid (6) is overlapped with the openings (5) closing them, and an open position in which the openings (5) are accessible for inserting waste into the container (1).

A handle (7) for handling and transporting the container (1), is pivotally coupled with the lid (6).

The container (1) has a pipe (8) placed inside the container (1) and arranged to fluidly communicate the container interior (4) with the exterior of the container (1), such that a disinfecting gas or liquid can circulate along the pipe (8) into the container to disinfect waste.

In this preferred embodiment, the pipe (8) is straight and has one end (8a) joined to the bottom base (3a), and a free end (8b) adjacent or near the upper base (2).

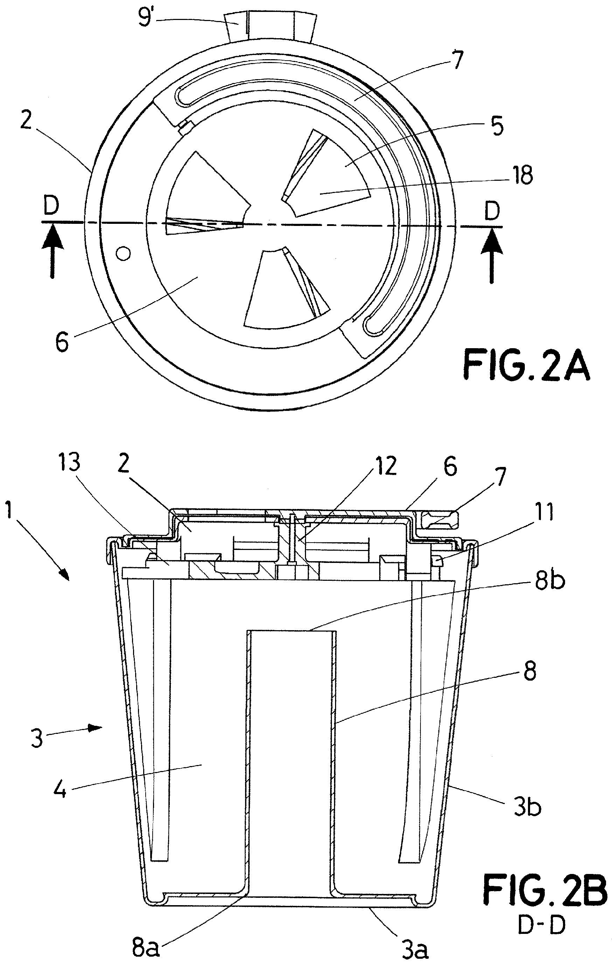

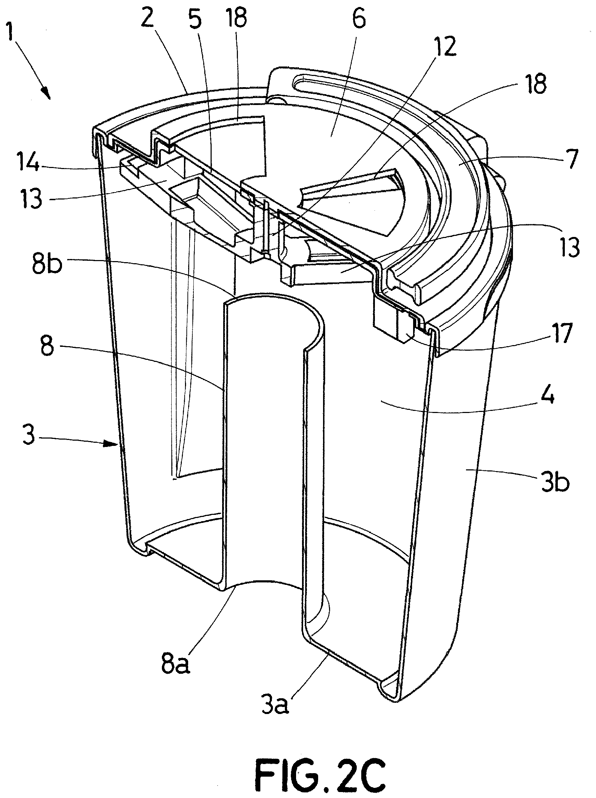

As shown specially in **Figures 2A, 2B** the openings (5) of the upper base (2) are arranged relative to the free end (8b) of the pipe (8), such that waste introduced through any of the openings (5) would be received in the container interior (4), that is, waste cannot fall inside the pipe through its open free end (8b). This is realized by having the openings (5) overlapping only with the bottom base (3a) of the bucket (3), and not overlapping with the open free end (8b). In addition, the height of the pipe (8) is designed such that the free end (8b) of the pipe, is close to the lower surface of the upper base (2).

The upper base (4) is movable with respect to the bucket (3) to allow extraction of the content of the container. Preferably, the container (1) has a hinge (9) connecting the upper base (4) with the bucket (3).

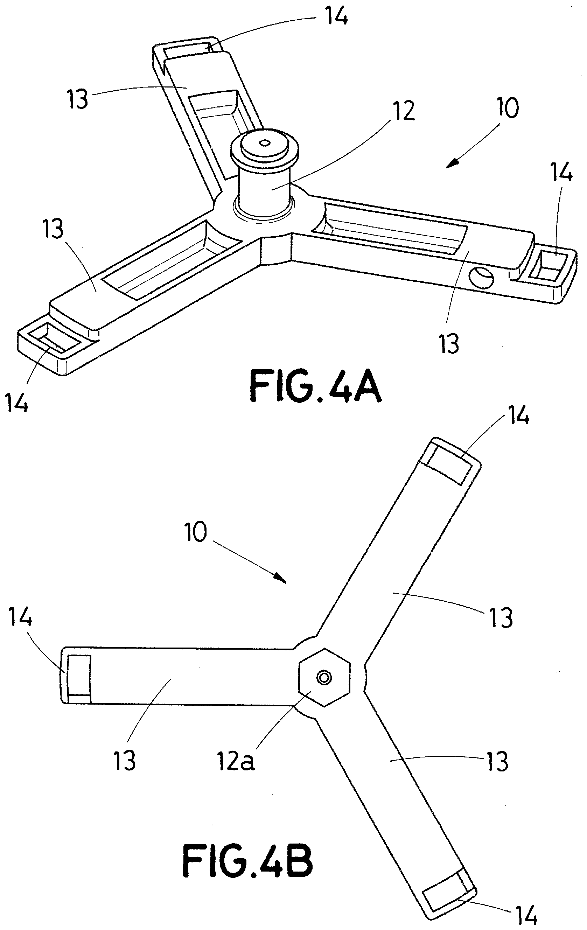

In addition, locking means (10) are provided and configured to be set in a locked position in which they attach the upper base (2) to the side wall (3b), and in a released position in which the upper base (2) can be pivoted with respect to the side wall (3b) by means of the hinge (9). The locking means (10) comprises first engaging means (3b) including hooks (11) attached to the side wall (3c ) as shown in **Figure 1A****,** and second engaging means that includes an axis (12) rotatably inserted in an opening 15) of the upper base (2), and two or more arms (13) radially extending from the axis (12), as represented in **Figure 4****.** Preferably, there are three arms (13) separated at 120° in a start arrangement.

The arms (13) are provided with loops (14) at their free ends, and are configured in a way that the hooks (11) of the first engaging means can be inserted in the loops (14) in a way that the first and second engaging means can engage and disengage with each other to attach or release the upper base (2) from the bucket (3), by rotating the axis (12).

The angular displacement of the arms (13), is limited by pairs of walls or stoppers (17), such that an arm (13) is placed in between the walls (17) of each pair so that the rotation in both directions of the second engaging means, is limited to facilitate the engagement and disengagement operation.

The second engaging means, in particular the axis (12), is accessible from outside the container through the pipe (8) to be rotated, to be set either in the locked position or in the released position. The pipe (8) and the axis (12) are co-axially arranged.

For that purpose, a tool (16) is provided such that it can be inserted from outside the container (1) through the pipe (8), to reach the axis (12). One end (16a) of the tool (16) can be inserted into a recess (12a) of the axis (12) and to engage with each other, such that the axis (12) can be rotated by rotating the tool (16) from outside the container (1).

For disinfecting the content of the container (1), this is placed upside down with the openings (5) the closed position, such that the end (8a) of the pipe, is placed at the top to be accessible. Then, steam of other disinfecting gas, is circulated through the pipe to fill the container interior (4).

Concerning the fabrication process, all the components of the container (1) described above can be manufactured from a thermoplastic material. As shown in **Figures 2A** and 2B, the bucket (3) and the pipe (8), preferably, are manufactured as an integral unitary body.

Other preferred embodiments of the present invention are described in the appended dependent claims and the multiple combinations of those claims.

## Claims

1. Medical waste container (1), comprising:
an upper base (2), a bottom base (3a) and a side wall (3b) joined to the bottom base (3a) to define the container interior (4) for containing waste,
at least one opening (5) for introducing waste into the container (1),
closing means for closing the at least one opening (5),
a pipe (8) placed inside the container and arranged to fluidly communicate the interior (4) of the container with the exterior of the container, such that a disinfecting gas or liquid can circulate along the pipe (8) into the container, and
wherein the at least one opening (5) is arranged relative to the pipe (8) such that waste introduced through the opening (5) would be received at the container interior (4).

2. Container according to claim 1, wherein the pipe (8) has two open ends, one open end (8a) joined to the bottom base (3a), to the upper base (2), or to the side wall (3b), and the other end is a free end (8b) which is placed inside the container (1).

3. Container according to claim 1 or 2, wherein the pipe (8) is straight and has one end (8a) joined to the bottom base (3a), and wherein the free end (8b) is placed below and adjacent to the upper base (2).

4. Container according to claim 2 or 3, wherein the at least one opening (5) does not overlap with the free end (8b) of the pipe (8).

5. Container according to any of the preceding claims, wherein the upper base (2) is movable with respect to the side wall (3b) to allow extraction of the content of the container (1), and wherein the at least one opening (5) is provided in the upper base (2).

6. Container according to any of the preceding claims, further comprising locking means (10) configured to be set in a locked position in which they attach the upper base (2) to the side wall (3b), and in a released position in which the upper base (2) can be moved with respect to the side wall (3b).

7. Container according to claim 6, wherein the locking means (10) are accessible through the pipe (8) to be set either in the locked position or in the released position.

8. Container according to any of the preceding claims, wherein the closing means for closing the at least one opening (5), comprises a lid (6) mounted on the upper base (2), and wherein the lid (6) is rotatable on the upper base (2) between a closed position in which the lid is overlapped with the opening (5) closing it, and an open position in which the opening (5) is accessible for inserting waste into the container.

9. Container according to any of the preceding claims, wherein the side wall (3b) has a free edge (3c) having a circumferential shape, and wherein the upper base (2) has a circular configuration and it is couplable to the free edge of the side wall.

10. Container according to any of the claims 6 to 9, wherein the locking means (10) comprises first engaging means attached to the side wall, and second engaging means coupled or couplable with the upper base (2), wherein the first and second engaging means are configured to engage and disengage with each other to attach or release the upper base (2) with the side wall (3b).

11. Container according to claim 10, wherein the first engaging means are fixed to an internal surface of the side wall (3b), and wherein the second engaging means are coupled to the upper base (2) and are rotatable with respect to the first engaging means, and wherein the first and second engaging means are configured to engage and disengage with each other upon rotation of the second engaging means.

12. Container according to claim 10 or 11, wherein the upper base (2) is disc-shaped and it has upper and lower surfaces, and wherein the lid (6) is mounted on the upper surface of the upper base, and the second engaging means are mounted on the lower surface of the upper base (2), and wherein the second engaging means are accessible from outside the container (1) through the pipe (8) to be rotated.

13. Container according to any of the claims 10 to 12, wherein the first engaging means includes hooks (11), and the second engaging means include an axis (12) rotatably coupled to the upper base (2) and two or more arms (13) radially extending from the axis (12), and wherein the arms (13) are provided with loops (14) at their free ends and are configured in a way that the hooks (11) of the first engaging means can be inserted in the loops (14) upon rotation of the second engaging means.

14. Container according to any of the claims 6 to 13, further comprising a tool (16) configured to be inserted from outside the container (1) through the pipe (8) and reach the second engaging means, and wherein one end (16a) of the tool and the second engaging means are configured to engage with each other, such that the second engaging means can be rotated by rotating the tool (16).
